# EUROPEAN PATENT APPLICATION

(11) **EP 3 308 718 A1**
(43) Date of publication of application: **18.04.2018**
(21) Application number: 16807306.2
(22) Date of filing: 27.05.2016
(51) Int. Cl.: A61B 10/02

(54) **BIOPSY NEEDLE**

(30) Priority: 10.06.2015 JP 2015117605
(71) Applicant: Olympus Corporation, Hachioji-shi Tokyo 192-8507 (JP)
(72) Inventor: NISHINA, Kenichi, Tokyo 192-8507 (JP); IIJIMA, Yasuhiro, Tokyo 192-8507 (JP)
(74) Representative: Schicker, Silvia
(86) International application number: PCT/JP2016/065761
(87) International publication number: WO 2016/199599

(57) **Abstract**

A biopsy needle (100) according to the present invention includes a tubular outer needle (110) having a needle tip (111) at one end in a longitudinal direction; and a pillar-shaped inner needle (120) configured to be inserted into and movable in the outer needle (110) in the longitudinal direction of the outer needle (110). The inner needle (120) includes: a distal end portion having a needle tip (121) at a distal end and having an inclined surface (122) inclined toward the needle tip (121) at the distal end; a notch portion (123) including a notch on a side surface closer to a proximal end side of the inner needle than the inclined surface (122) to collect body tissues; and a groove (124) leading from the distal end portion to the notch portion (123).

## Description

### Field

The present invention relates to a biopsy needle for collecting body tissues.

### Background

Conventionally, biopsy for collecting body tissues is performed for a pathological diagnostic confirmation by puncturing lesion tissue by introducing an elongated biopsy needle to an observed region through a channel for treatment tools for an ultrasound endoscope, using ultrasonic tomogram obtained by an ultrasound endoscope being used as a guide. As a biopsy needle, there is a proposed configuration in which an inner needle having a cutout (notch) for tissue collection formed on a distal end-side side surface is arranged inside a hollow outer needle and the inner needle and the outer needle are configured to advance or retreat by a moving mechanism (for example, refer to Patent Literature 1). In the biopsy, puncture is performed with the biopsy needle to reach the tissue of the biopsy site in a state where the outer needle of the biopsy needle substantially covers the notch of the inner needle, and thereafter, the inner needle is allowed to protrude from the distal end of the outer needle until the notch is exposed. This causes the body tissue to enter the inside of the notch, and thus, while the body tissue that has entered the inside of the notch is cut with the distal end of the outer needle by allowing the outer needle to advance, the notch is covered with the outer needle with the body tissue caught inside the notch. In this state, the biopsy needle is pulled out from the biopsy site, and thereafter, the notch is exposed by retreating the outer needle, and the body tissue inside the notch is collected.

### Citation List

### Patent Literature

Patent Literature 1: JP-T 2009-531115

### Summary

### Technical Problem

While biopsy for the diagnosis of prostate cancer is currently performed transrectally or transperineally, it is considered desirable to achieve a method for performing biopsy from the inside of the prostate transurethrally in order to reduce invasion to a patient. According to this method, a needle tip would not come in contact with the nerve running on the outside of the prostate as long as a capsule (outer membrane) of the prostate is not penetrated from the inside to the outside, and thus, the burden on the patient can be further reduced.

With the conventional biopsy needle, however, a notch capable of collecting a specimen comes at a proximal end-side section located about 5 mm from the needle tip. Therefore, when the notch is brought close to the capsule boundary in order to collect tissue of the prostate near the capsule boundary, there might be a case of penetrating the capsule by the needle tip and there is no denying the possibility of the needle tip coming in contact with the nerve. Therefore, there was a demand for capability of collecting body tissue in the vicinity of the capsule boundary with substantially no exposure of the needle tip of the biopsy needle from the capsule of the prostate to the outside of the capsule.

The present invention has been made in view of the foregoing, and an object of the invention is to provide a biopsy needle capable of reliably collecting tissues and reducing the length of the biopsy needle protruding from a tissue collection target site at the time of tissue collection.

### Solution to Problem

In order to solve the above described problem and achieve the object, a biopsy needle according to the invention includes an outer needle being tubular and having a first needle tip at one end of the outer needle in a longitudinal direction, and an inner needle having a pillar shape and configured to be inserted into and movable in the outer needle in the longitudinal direction of the outer needle. The inner needle includes: a distal end portion having a second needle tip at a distal end of the inner needle and having an inclined surface inclined toward the second needle tip at the distal end; a notch portion having a notch on a side surface closer to a proximal end side of the inner needle than the inclined surface to collect body tissues; and a guide portion leading from the distal end portion to the notch portion.

In the biopsy needle according to the invention, the guide portion is a groove leading from the distal end portion to the notch portion.

In the biopsy needle according to the invention, a depth of the groove is shallower at the distal end portion than at a connection between the guide portion and the notch portion.

In the biopsy needle according to the invention, a bottom of the groove is inclined from the distal end portion toward the connection.

In the biopsy needle according to the invention, the guide portion is a cutout portion extending from the distal end portion to the notch portion in a longitudinal direction of the inner needle.

In the biopsy needle according to the invention, a cutout surface of the cutout portion is inclined from the distal end portion toward the connection.

In the biopsy needle according to the invention, the notch portion has a rib.

In the biopsy needle according to the invention, an upper portion of the rib is cut out in a longitudinal direction of the inner needle.

In the biopsy needle according to the invention, the notch portion and the guide portion are located on a same side as the second needle tip in a transverse direction of the inner needle.

In the biopsy needle according to the invention, the notch portion and the guide portion are located on an opposite side to the second needle tip in a transverse direction of the inner needle.

In the biopsy needle according to the invention, a distal end of the outer needle is obliquely cut out to form the first needle tip. The inclined surface of the inner needle and a cutout at the distal end of the outer needle are substantially parallel to each other.

The biopsy needle according to the invention further includes a moving mechanism configured to slide the outer needle and the inner needle in the longitudinal direction independently of each other such that a maximum slidable distance of the outer needle in a distal end direction is larger than a maximum slidable distance of the inner needle in a distal end direction. The moving mechanism is configured to move the outer needle and the inner needle so as to switch between a first state in which the second needle tip is located on more distal end side than the first needle tip and a second state in which the first needle tip is located on more distal end side than the second needle tip.

In the biopsy needle according to the invention, the inner needle is columnar.

### Advantageous Effects of Invention

A biopsy needle according to the present invention includes an outer needle being tubular and having a first needle tip at one end of the outer needle in a longitudinal direction, and an inner needle having a pillar shape and configured to be inserted into and movable in the outer needle in the longitudinal direction of the outer needle. The inner needle includes a distal end portion having a second needle tip at a distal end of the inner needle and having an inclined surface inclined toward the second needle tip at the distal end, a notch portion having a notch on a side surface closer to a proximal end side of the inner needle than the inclined surface to collect body tissues, and a guide portion leading from the distal end portion to the notch portion. With this structure, it is possible to reliably collect the tissues via the guide portion. Since the guide portion leads to the distal end portion, it is possible to reduce a length of the biopsy needle protruding from a tissue collection target site at the time of collecting the tissues.

### Brief Description of Drawings

FIG. 1 is a diagram for illustrating a distal end section of a biopsy needle according to a first embodiment.
FIG. 2 is a cross-sectional view taken along line A-A in FIG. 1.
FIG. 3 is a cross-sectional view taken along line B-B in FIG. 1.
FIG. 4 is a cross-sectional view of the distal end section of the biopsy needle illustrated in FIG. 1 after the body tissue collection, taken along a plane passing through the center axis of the biopsy needle in the longitudinal direction and the center of the needle tip of an inner needle.
FIG. 5 is a diagram for illustrating a biopsy method implemented by the biopsy needle illustrated in FIG. 1.
FIG. 6 is a diagram for illustrating a distal end section of a biopsy needle according to a conventional technique.
FIG. 7 is a cross-sectional view taken along line C-C in FIG. 6.
FIG. 8 is a cross-sectional view of the distal end section of the biopsy needle according to the conventional technique after the body tissue is collected, taken along a plane passing through the center axis of the biopsy needle in the longitudinal direction and the center of the needle tip of the inner needle.
FIG. 9 is a cross-sectional view of the distal end section of the biopsy needle according to the conventional technique after the body tissue is collected, taken along a plane passing through the center axis of the biopsy needle in the longitudinal direction and the center of the needle tip of the inner needle.
FIG. 10 is a cross-sectional view of the distal end section of the biopsy needle according to the conventional technique after the body tissue is collected, taken along a plane passing through the center axis of the biopsy needle in the longitudinal direction and the center of the needle tip of the inner needle.
FIG. 11 is a cross-sectional view of the biopsy needle according to the first embodiment, taken along a plane passing through the center axis of the biopsy needle in the longitudinal direction and the center of the needle tip of the inner needle.
FIG. 12 is a diagram illustrating a first state and a second state of the biopsy needle illustrated in FIG. 1.
FIG. 13 is a diagram illustrating protruding operation of the outer needle and the inner needle in the biopsy needle illustrated in FIG. 1.
FIG. 14A is a diagram illustrating biopsy operation of the biopsy needle illustrated in FIG. 11.
FIG. 14B is a diagram illustrating biopsy operation of the biopsy needle illustrated in FIG. 11.
FIG. 14C is a diagram illustrating biopsy operation of the biopsy needle illustrated in FIG. 11.
FIG. 14D is a diagram illustrating biopsy operation of the biopsy needle illustrated in FIG. 11.
FIG. 14E is a diagram illustrating biopsy operation of the biopsy needle illustrated in FIG. 11.
FIG. 14F is a diagram illustrating biopsy operation of the biopsy needle illustrated in FIG. 11.
FIG. 15 is a cross-sectional view of a distal end section of a biopsy needle according to a first modification of the first embodiment, taken along a plane passing through the center axis of the biopsy needle in the longitudinal direction.
FIG. 16 is a cross-sectional view taken along line D-D in FIG. 15.
FIG. 17 is a plan view of a distal end section of a biopsy needle according to a second modification of the first embodiment.
FIG. 18 is a cross-sectional view of a distal end section of a biopsy needle according to the second modification of the first embodiment, taken along a plane passing through the center axis of the biopsy needle in the longitudinal direction.
FIG. 19 is a diagram for illustrating a distal end section of a biopsy needle according to a second embodiment.
FIG. 20 is a cross-sectional view of the distal end section of the biopsy needle according to the second embodiment after body tissue collection, taken along a plane passing through the center axis of the biopsy needle in the longitudinal direction.
FIG. 21 is a diagram for illustrating a distal end section of a biopsy needle according to a first modification of the second embodiment.
FIG. 22 is a perspective view of a distal end portion of the inner needle illustrated in FIG. 21.
FIG. 23 is a cross-sectional view taken along line E-E in FIG. 21.
FIG. 24 is a plan view of a distal end section of another biopsy needle according to the first modification of the second embodiment.
FIG. 25 is a diagram for illustrating a distal end section of a biopsy needle according to a second modification of the second embodiment.
FIG. 26 is a schematic view illustrating another example of the distal end section of the biopsy needle according to the second modification of the second embodiment.
FIG. 27 is a cross-sectional view taken along line F-F in FIG. 26.
FIG. 28 is a schematic view illustrating another example of the distal end section of the biopsy needle according to the second modification of the second embodiment.
FIG. 29 is a cross-sectional view of a distal end section of a biopsy needle according to a third embodiment, taken along a plane passing through the center axis of the biopsy needle in the longitudinal direction.
FIG. 30 is a cross-sectional view taken along line G-G in FIG. 29.
FIG. 31 is a cross-sectional view of a distal end section of a biopsy needle according to a first modification of the third embodiment, taken along a plane passing through the center axis of the biopsy needle in the longitudinal direction.
FIG. 32 is a cross-sectional view taken along line H-H in FIG. 31.
FIG. 33 is a side view of a distal end section of an inner needle according to a second modification of the third embodiment.
FIG. 34 is a perspective view of the distal end section of the inner needle illustrated in FIG. 33.
FIG. 35 is a cross-sectional view taken along line I-I in FIG. 33.
FIG. 36 is a cross-sectional view of a distal end section of another inner needle according to the second modification of the third embodiment, taken along a plane passing through the center axis of the inner needle in the longitudinal direction.
FIG. 37 is a perspective view of the distal end section of the inner needle illustrated in FIG. 36.
FIG. 38 is a cross-sectional view taken along line J-J in FIG. 36.
FIG. 39 is a diagram for illustrating a distal end section of a biopsy needle according to a fourth embodiment.
FIG. 40 is a schematic view in the direction of arrow L in FIG. 39.
FIG. 41 is a cross-sectional view taken along line K-K in FIG. 39.
FIG. 42 is a diagram for illustrating a distal end section of another biopsy needle according to the fourth embodiment.
FIG. 43 is a schematic view in the direction of arrow N in FIG. 42.
FIG. 44 is a cross-sectional view taken along line M-M in FIG. 42.
FIG. 45 is a diagram for illustrating a distal end section of another biopsy needle according to the fourth embodiment.
FIG. 46 is a schematic view in the direction of arrow P in FIG. 45.
FIG. 47 is a cross-sectional view taken along line O-O in FIG. 45.

### Description of Embodiments

Embodiments of the present invention will be described below in detail with reference to the drawings. The present invention is not limited to the following embodiments. The drawings referred to in the following description merely schematically illustrate the shapes, sizes, and positional relations to such degrees that the contents of the present invention are understandable. Accordingly, the present invention is not limited only to the shapes, sizes, and positional relations exemplified in the individual drawings. While the following description exemplifies a biopsy needle configured to puncture the interior of body tissue of an animal including a human and to collect the body tissue, the present invention is not limited by the embodiments. The same reference signs are used to designate the same elements throughout the drawings.

### (First Embodiment)

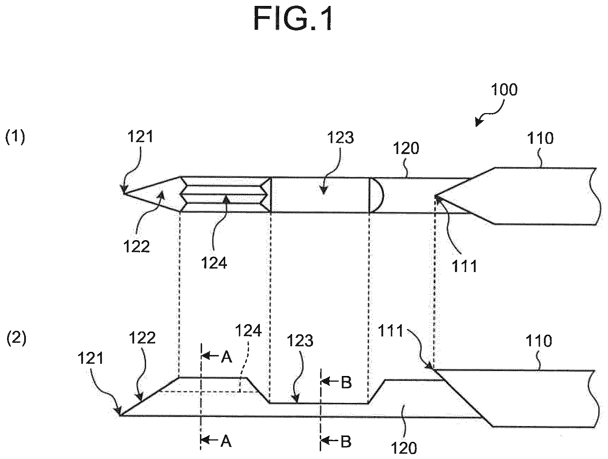

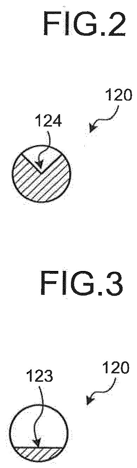

FIG. 1 is a diagram for illustrating a distal end section of a biopsy needle according to a first embodiment.
(1) of FIG. 1 is a plan view of the distal end section of the biopsy needle according to the first embodiment, and
(2) of FIG. 1 is a side view of the distal end section of the biopsy needle according to the first embodiment. FIG. 2 is a cross-sectional view taken along line A-A in FIG. 1. FIG. 3 is a cross-sectional view taken along line B-B in FIG. 1.

As illustrated in FIGS. 1 to 3, a biopsy needle 100 according to the first embodiment includes a tubular outer needle 110 and an inner needle 120 that is solid and columnar. The outer needle 110 extends in a longitudinal direction. The inner needle 120 is configured to be inserted into and movable in the outer needle 110 in the longitudinal direction of the outer needle. As described below, the biopsy needle 100 further includes an operating unit (not illustrated) internally housing a proximal end of the outer needle 110 and the inner needle 120 and having a moving mechanism for sliding the outer needle 110 and the inner needle 120 independently in the longitudinal direction.

The outer needle 110 has a tubular shape with a pointed distal end, and includes a needle tip 111 (first needle tip) at the distal end in the longitudinal direction. The outer needle 110 is formed of a biocompatible material, for example, metal such as stainless steel, titanium, aluminum, or a resin such as a fluororesin.

Similarly to the outer needle 110, the inner needle 120 is formed of a biocompatible material and includes a distal end portion and a notch portion 123. The distal end portion includes a needle tip 121 (second needle tip) and an inclined surface 122. The needle tip 121 is formed at the distal end of the inner needle. The inclined surface 122 is inclined toward the needle tip 121 at the distal end. The notch portion 123 includes a notch for collecting body tissue formed on the side surface on more proximal end side compared with the inclined surface 122. The needle tip 121 is located on a plane passing through the center axis of the inner needle 120 in the longitudinal direction. When viewing in the transverse direction, the distal end of the inner needle 120 is cut so as to form a top portion on an opposite side of the inclined surface 122, whereby the needle tip 121 is tapered. The inclined surface 122 is formed by a processing method such as a lancet, a back cut, a ceramic set, or flat grinding. In the notch portion 123, a notch is formed by cutting out the columnar portion along the longitudinal direction so as to form a cutout surface into a flat surface at a section on more proximal end side compared with the inclined surface 122 (refer to FIG. 3). In the notch portion 123, the distal end surface and the proximal end surface are inclined from the bottom surface toward the upper portion of the notch portion 123 in side view. The inner needle 120 may have a prismatic shape in addition to a columnar shape.

Furthermore, the inner needle 120 includes a groove 124 (guide portion) communicating from the distal end portion to the notch portion 123. As illustrated in FIGS. 1 and 2, the groove 124 is formed by cutting on the inner needle 120 in a V shape beginning from the proximal end of the inclined surface 122 to the distal end surface of the notch portion 123. In the examples of FIGS. 1 to 3, the depth of the groove 124 is set to be shallower than the depth of the notch portion 123. The notch portion 123 and the groove 124 are located on the side opposite to the formation portion (top portion) of the needle tip 121 in the transverse direction.

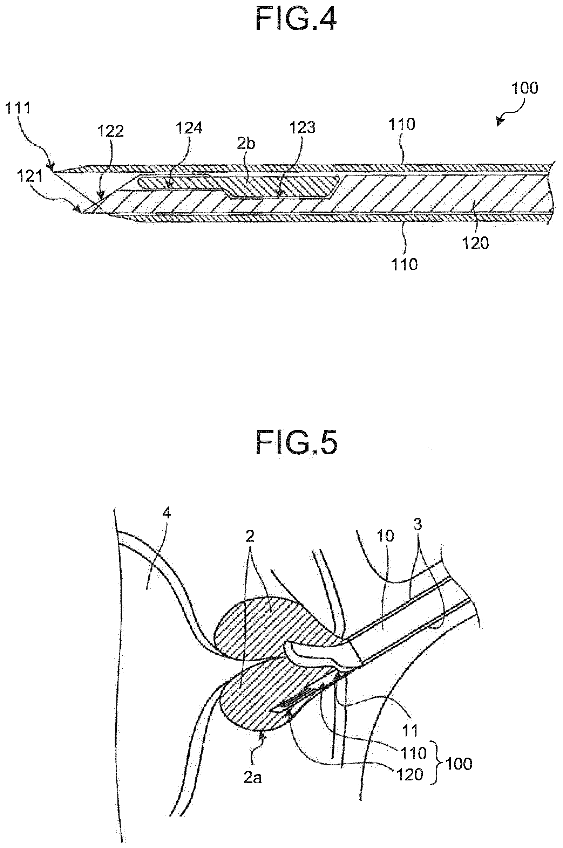

FIG. 4 is a cross-sectional view of the distal end section of the biopsy needle 100 after the body tissue collection, taken along a plane passing through the center axis of the biopsy needle 100 in the longitudinal direction and the center of the needle tip 121 of the inner needle 120. When body tissue is punctured, the body tissue naturally enters the groove 124 on the distal end side by energy at the time of ejection of the inner needle 120, and by further ejecting the inner needle 120, the body tissue is introduced to the notch portion 123 on more proximal end side compared with the groove 124, via the groove 124. Moreover, even in a case where the notch portion 123 has not reached a body tissue collection target region, the collection target body tissue can be accommodated in the groove 124 as long as the groove 124 has reached the body tissue collection target region. As illustrated in FIG. 4, the biopsy needle 100 can accommodate body tissue 2b in both the distal end-side groove 124 and the proximal end-side notch portion 123. Accordingly, it is possible to collect a large amount of body tissue compared with the configuration without the groove 124.

FIG. 5 is a diagram for illustrating a biopsy method implemented by the biopsy needle 100 illustrated in FIG. 1. As illustrated in FIG. 5, using the ultrasonic tomogram by an ultrasound endoscope as a guide, an insertion unit 10 of the ultrasound endoscope is allowed to reach a prostate 2 as a biopsy target via a urethra 3 of the patient. The distal end of the biopsy needle inserted into the treatment tool channel of the insertion unit 10 is caused to protrude from an opening 11 at the distal end of the insertion unit 10, and the prostate 2 is punctured by the outer needle 110 and the inner needle 120. In this case, the distal end of the inner needle 120 is caused to protrude from the outer needle 110 until at least the groove 124 is exposed. With protrusion of the inner needle 120, body tissue is sequentially introduced via the groove 124 to the notch portion 123 on more proximal end side compared with the groove 124. Thereafter, the outer needle 110 is caused to advance in the distal end direction, and while the body tissue that has entered the groove 124 and the notch portion 123 is cut by the distal end of the outer needle 110, the groove 124 and the notch portion 123 are covered with the outer needle 110 in a state where the body tissue is caught inside the groove 124 and the notch portion 123. In this state, the biopsy needle is pulled out of the body via the treatment tool channel, and thereafter, the body tissue caught in the groove 124 and the notch portion 123 is collected. A bladder 4 is located behind the prostate 2.

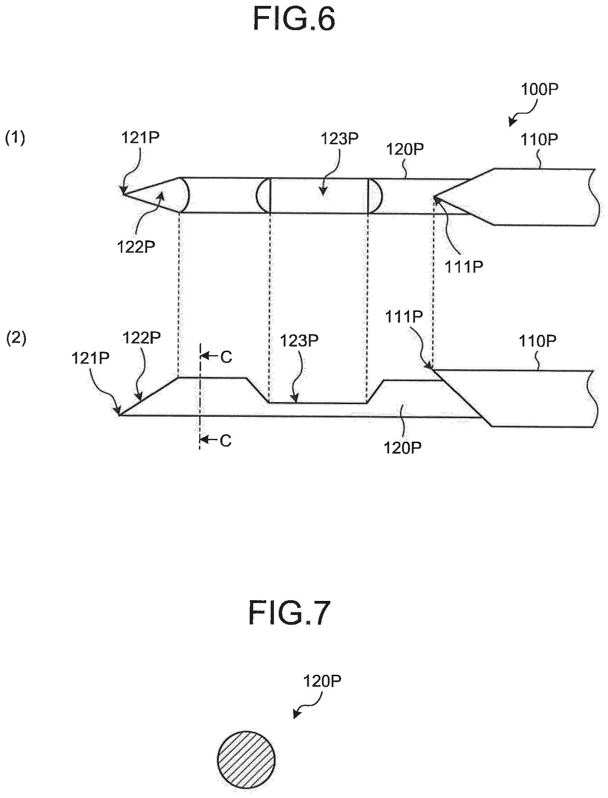

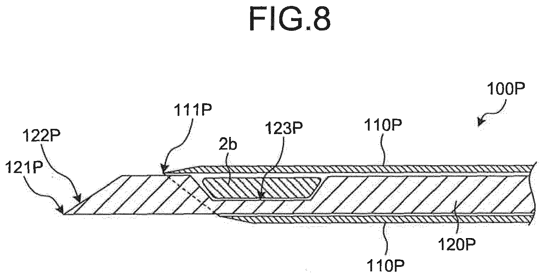

A conventional biopsy needle will now be described. FIG. 6 is a diagram for illustrating a distal end section of a biopsy needle according to a conventional technique. (1) of FIG. 6 is a plan view of the distal end section of the biopsy needle according to the conventional technique, and (2) of FIG. 6 is a side view of the distal end section of the biopsy needle according to the conventional technique. FIG. 7 is a cross-sectional view taken along line C-C in FIG. 6. FIGS. 8 to 10 are cross-sectional views of the distal end section of the biopsy needle according to the conventional technique after the body tissue collection, taken along a plane passing through the center axis of the biopsy needle in the longitudinal direction and the center of the needle tip of the inner needle.

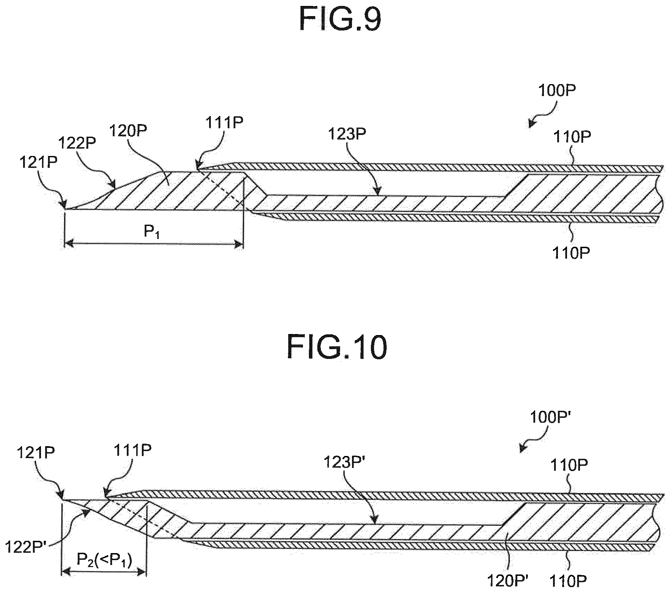

As illustrated in FIGS. 6 and 7, a conventional biopsy needle 100P is configured, in order to collect body tissue, to include solely a notch portion 123P obtained by cutting out the side surface on the distal end side of an inner needle 120P so as to obtain a semicircular cross section. Accordingly, the region between the proximal end of an inclined surface 122P and the notch portion 123P has a solid cylindrical shape (refer to FIG. 7). Therefore, in a case where the biopsy needle 100P is used, there is a need to cause the inner needle 120P to protrude from an outer needle 110P until the notch portion 123P is exposed, in order to collect body tissue 2b into the notch portion 123P as illustrated in FIG. 8. In addition, in the conventional biopsy needle 100P, in order to reliably cover a portion up to the distal end of the notch portion 123P with the outer needle 110P so as not to allow the body tissue to be released from the notch portion 123P after the body tissue collection, there is a need, as illustrated in FIG. 9, to allow a certain margin by increasing a length P₁ from a needle tip 121P of the inner needle 120P to the notch portion 123P to a length of about 5 to 6 mm. For this reason, with the conventional biopsy needle 100P, there is a need to puncture to reach a depth 5 to 6 mm deeper than the depth of the site including the tissue to be collected with the needle tip 121P of the inner needle 120P. Moreover, as an attempt to perform tissue collection without inserting the needle deeply into the tissue, as in a biopsy needle 100P' illustrated in FIG. 10, there is an examined configuration in which a notch portion 123P' is extended in the distal end direction to the vicinity of the proximal end of an inclined surface 122P', having a length P₂ of an inner needle 120P' protruding from the tissue collection target site being reduced to about 2 to 3 mm. In the configuration illustrated in FIG. 10, however, the notch portion 123P' extends to the vicinity of the proximal end of the inclined surface 122P', and this might reduce the strength of the distal end portion of the inner needle 120P', leading to bending of the inner needle 120P' in a case where the inner needle 120P' punctures hard tissue.

In contrast, the inner needle 120 according to the first embodiment merely includes the groove 124 communicating from the distal end portion to the notch portion 123, with the notch portion 123 being arranged at a same position. This configuration makes it possible to maintain the strength of the portion from the distal end portion to the notch portion 123 and to easily transmit puncture force to the needle tip 121, and thus, can avoid bending of the inner needle 120 even when the inner needle 120 punctures the hard tissue.

Moreover, in the first embodiment, the groove 124 leading from the distal end portion to the notch portion 123 is formed, and the body tissue can be collected when at least the groove 124 reaches the body tissue collection target region. Therefore, according to the first embodiment, it is sufficient, at the time of biopsy, that the groove 124 on more distal end side compared with the notch portion 123 reaches the body tissue collection target region, and there is no need to cause the inner needle 120 to protrude to a degree to allow the notch portion 123 on the proximal end side to reach the inner needle 120. Accordingly, it is possible to collect the body tissue in the vicinity of the needle tip 121 into the groove 124 and the notch portion 123 while reducing the protruding length of the inner needle 120. Moreover, according to the first embodiment, the body tissue 2b can be accommodated in both the distal end-side groove 124 and the proximal end-side notch portion 123, and thus, a large amount of body tissue can be collected.

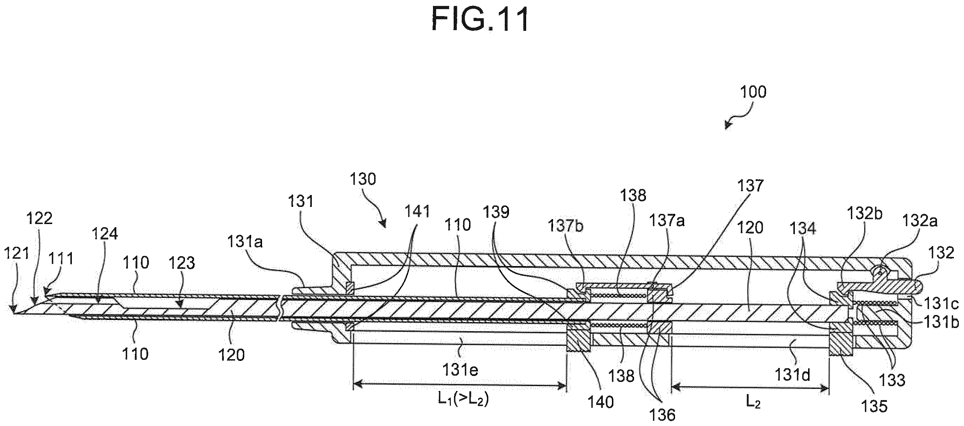

Next, an overall configuration of the biopsy needle 100 will be described. FIG. 11 is a cross-sectional view of the biopsy needle 100, taken along a plane passing through the center axis of the biopsy needle 100 in the longitudinal direction and the center of the needle tip of the inner needle 120. As illustrated in FIG. 11, in addition to the outer needle 110 and the inner needle 120, the biopsy needle 100 further includes an operating unit 130 internally housing proximal ends of the outer needle 110 and the inner needle 120 and having a moving mechanism for sliding the outer needle 110 and the inner needle 120 independently in the longitudinal direction.

The operating unit 130 has a configuration with portions to achieve a function as a moving mechanism, including a trigger button 132, an inner needle charging coil spring 133, an inner needle slider 134, an inner needle knob 135, an inner needle stopper 136, an outer needle fixing hook release lever 137, an outer needle charging coil spring 138, an outer needle slider 139, an outer needle knob 140, and an outer needle stopper 141, being assembled onto a hollow and pillar-shaped operating unit main body 131.

The operating unit main body 131 includes: a Luer fitting portion 131a provided at the distal end; a spring assembly protrusion 131b provided at the inner proximal end; a trigger button hole 131c provided at the upper portion of the spring assembly protrusion 131b; an inner needle knob groove 131d dug in the longitudinal direction provided at the proximal end-side bottom; and an outer needle knob groove 131e dug in the longitudinal direction provided at the distal end-side bottom. The distal ends of the outer needle 110 and the inner needle 120 protrude from the Luer fitting portion 131a in the distal end direction. A proximal end of an inner needle charging coil spring 133 described below is assembled to the spring assembly protrusion 131b. A proximal end of a trigger button 132 described below protrudes from the trigger button hole 131c. An inner needle knob 135 described below is slidable in the longitudinal direction in the inner needle knob groove 131d. An outer needle knob 140 described below is slidable in the longitudinal direction in the outer needle knob groove 131e.

The trigger button 132 serves as a trigger for advancing operation of the inner needle 120, and both ends alternately rise and fall about a fulcrum 132a connected to the inside of the operating unit main body 131. An inner needle fixing hook 132b to be caught in a recess of the inner needle slider 134 described below is provided at the distal end of the trigger button 132.

The inner needle charging coil spring 133 has its proximal end being assembled to a proximal end portion of the spring assembly protrusion 131b and has its distal end being assembled to a proximal end-side surface of the inner needle slider 134 described below. The inner needle charging coil spring 133 is extended after compression (charging), thereby biasing the inner needle slider 134 toward the distal end direction.

The inner needle slider 134 is connected to the proximal end of the inner needle 120 and advances in the distal end direction by the biasing in the distal end direction by the inner needle charging coil spring 133, and together with this, causes the inner needle 120 to advance in the distal end direction. A recess into which the inner needle fixing hook 132b fits is formed on the upper surface of the inner needle slider 134.

The inner needle knob 135 is slidable along the inner needle knob groove 131d in the longitudinal direction. The upper surface of the inner needle knob 135 is connected to the bottom surface of the inner needle slider 134. The lower portion of the inner needle knob 135 protrudes from the inner needle knob groove 131d. The inner needle knob 135 advances in the inner needle knob groove 131d together with the advance of the inner needle slider 134. Moreover, an operator of the biopsy needle 100 can retreat the inner needle slider 134 and the inner needle 120 toward the proximal end side by retreating the inner needle knob 135 toward the proximal end side along the inner needle knob groove 131d. In a case where the inner needle knob 135 is retreated to the proximal end of the inner needle knob groove 131d, the inner needle fixing hook 132b fits into the recess of the inner needle slider 134, and the inner needle 120 is fixed at a position to be arranged at the most proximal end side.

The inner needle stopper 136 stops the advance operation of the inner needle slider 134, and together with this stops the advance operation of the inner needle 120.

Both ends of the outer needle fixing hook release lever 137 rise and fall about a fulcrum 137a connected to the inner needle stopper 136. An outer needle fixing hook 137b to be caught in a recess of the outer needle slider 139 described below is provided at the distal end of the outer needle fixing hook release lever 137.

The outer needle charging coil spring 138 has its proximal end being assembled to a distal end-side side surface of the inner needle stopper 136 and has its distal end being assembled to a proximal end-side side surface of the outer needle slider 139 described below, and extended after compression (charging), thereby biasing the outer needle slider 139 toward the distal end direction.

The outer needle slider 139 is connected to the proximal end of the outer needle 110 and advances in the distal end direction by the biasing in the distal end direction by the outer needle charging coil spring 138. Together with this, the outer needle slider 139 causes the outer needle 110 to advance in the distal end direction. A recess into which the outer needle fixing hook 137b fits is formed on the upper surface of the outer needle slider 139.

The outer needle knob 140 is slidable along the outer needle knob groove 131e in the longitudinal direction. The upper surface of the outer needle knob 140 is connected to the bottom surface of the outer needle slider 139. The lower portion of the outer needle knob 140 protrudes from the outer needle knob groove 131e. The outer needle knob 140 advances in the outer needle knob groove 131e together with the advance of the outer needle slider 139. Moreover, the operator of the biopsy needle 100 retreats the outer needle slider 139 and the outer needle 110 toward the proximal end side by retreating the outer needle knob 140 toward the proximal end side along the outer needle knob groove 131e In a case where the outer needle knob 140 is retreated to the proximal end of the outer needle knob groove 131e, the outer needle fixing hook 137b fits into the recess of the outer needle slider 139, and the outer needle 110 is fixed at a position where it is arranged at the most proximal end side.

The outer needle stopper 141 is provided at the distal end portion of the operating unit main body 131, stops the advance operation of the outer needle slider 139, and together with this stops the advance operation of the outer needle 110.

A length L₁ of the outer needle knob groove 131e in the longitudinal direction that is, the stroke (maximum slidable distance) L₁ of the outer needle 110 toward the distal end direction is set to be greater than a length L₂ of the inner needle knob groove 131d in the longitudinal direction, that is, a stroke L₂ of the inner needle 120 toward the distal end direction. The stroke L₂ is set so as to allow at least the groove 124 to be exposed from the needle tip 111 of the outer needle 110 to such a degree to enable tissue collection.

The biopsy needle 100 according to the present embodiment is configured to form the groove 124 leading from the distal end portion to the notch portion 123, to define a first state and a second state and allow switching between the first state and the second state, thereby reliably reducing the protruding length of the inner needle 120 from the collection target at the time of biopsy to avoid penetration of the capsule 2a of the prostate 2 from the inside to the outside by the needle tip of the inner needle 120, and enabling collection of the body tissue into the groove 124 and the notch portion 123. The biopsy needle 100 defines the first state and the second state by setting the longitudinal stroke L₁ of the outer needle 110 greater than the longitudinal stroke L₂ of the inner needle 120. Next, the first state and the second state of the biopsy needle 100 will be described.

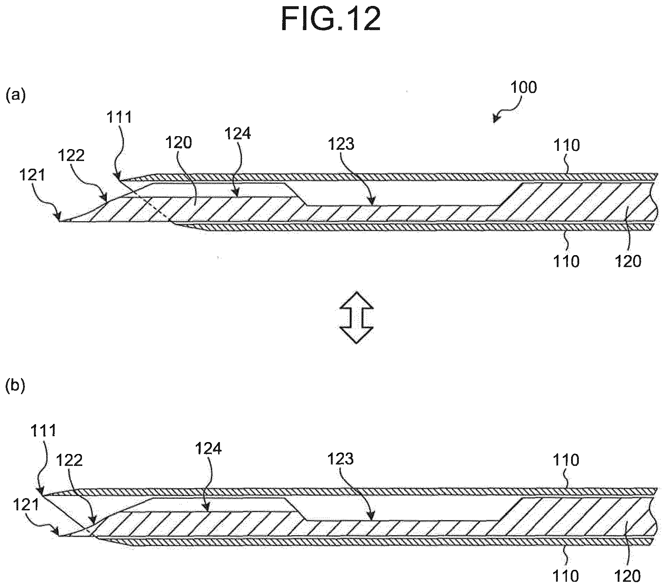

FIG. 12 is a diagram illustrating the first state and the second state of the biopsy needle 100 illustrated in FIG. 1, being a cross sectional view of the distal end section of the biopsy needle 100 taken along a plane passing through the center axis of the biopsy needle 100 in the longitudinal direction and passing through the needle tip 121 of the inner needle 120. FIG. 12 (a) is a diagram illustrating the first state. The first state is a charging state in which both the inner needle charging coil spring 133 and the outer needle charging coil spring 138 are compressed and charging energy for extension. In other words, the first state is a state of charging the energy for causing the outer needle 110 and the inner needle 120 to protrude, and the state in which the outer needle 110 and the inner needle 120 puncture the biopsy site. As illustrated in (a) of FIG. 12, the needle tip 121 of the inner needle 120 is located on more distal end side of the needle tip 111 compared with the outer needle 110 in the first state. Moreover, the needle tip 111 of the outer needle 110 is ideally located, in the first state, at more distal end side compared with the distal end of the groove 124 so as to prevent the body tissue other than the collection target from entering the groove 124 before reaching the collection target body tissue.

In FIG. 12, (b) is a diagram illustrating the second state of the biopsy needle 100, being a cross sectional view of the distal end section of the biopsy needle 100 taken along a plane passing through the center axis in the longitudinal direction and passing through the needle tip 121 of the inner needle 120. The second state is a state in which the inner needle 120 and the outer needle 110 have slid to the biopsy site in the distal end direction and the biopsy collection is completed. In the second state, the needle tip 111 of the outer needle 110 is located on more distal end side compared with the needle tip 121 of the inner needle 120. That is, the outer needle 110 covers the entire groove 124 of the inner needle 120.

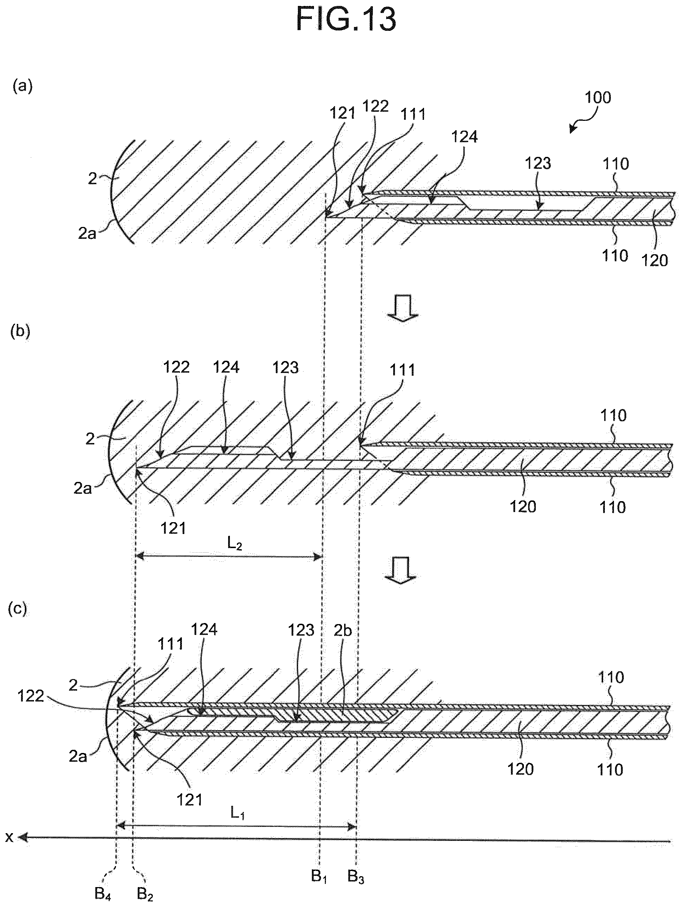

Next, FIG. 13 is a diagram illustrating the protruding operation of the outer needle 110 and the inner needle 120 in the biopsy needle 100 illustrated in FIG. 1. FIG. 13 illustrates a case where the inner needle 120 and the outer needle 110 protrude in the x-axis direction. Moreover, FIG. 13 is a cross-sectional view of the distal end section of the biopsy needle 100 taken along a plane passing through the center axis of the biopsy needle 100 in the longitudinal direction and passing through the needle tip 121 of the inner needle 120.

In FIG. 13, (a) illustrates the first state (charging state) in which the needle tip 121 of the inner needle 120 is located on more distal end side compared with the needle tip 111 of the outer needle 110, being the state before puncturing a site in the vicinity of the capsule 2a of the prostate 2 as a biopsy site.

In FIG. 13, (b) illustrates a state in which the inner needle 120 alone protrudes in the x-axis direction on the distal end side with the movement of the inner needle 120 by the moving mechanism to collect the tissue in the vicinity of the capsule 2a of the prostate 2. In this case, the inner needle 120 protrudes in the x-axis direction by the stroke L₂ as compared with the first state. Accordingly, the needle tip 121 of the inner needle 120 reaches a position B₂ in the close proximity of the capsule 2a, the position moved in the x-axis direction by the stroke L₂ from a position B₁ in the first state. With this configuration, the groove 124 is sufficiently exposed, and the tissue of the prostate 2 enters the groove 124. Furthermore, the tissue of the prostate 2 enters the notch portion 123 via the groove 124.

In FIG. 13, (c) illustrates a state in which the outer needle 110 has also protruded and the biopsy operation has been finished, that is, in the second state. The outer needle 110 protrudes more in the x-axis direction by the stroke L₁ compared with the first state. This stroke L₁ is greater than the stroke L₂. Accordingly, the needle tip 111 of the outer needle 110 located at a position B₃ on more proximal end side compared with the needle tip 121 of the inner needle 120 in the first state exceeds the notch portion 123 and the groove 124 of the inner needle 120, and then, reaches a position B₄ beyond the needle tip 121 of the inner needle 120. By the protrusion of the outer needle 110, while the tissue of the prostate 2 that has entered the notch portion 123 and the groove 124 is cut by the needle tip 111 of the outer needle 110, the notch portion 123 and the groove 124 can be completely covered with the outer needle 110 in a state where the body tissue 2b is caught inside the notch portion 123 and the groove 124.

In this manner, in the biopsy needle 100 according to the present embodiment, by setting the longitudinal stroke L₁ of the outer needle 110 greater than the longitudinal stroke L₂ of the inner needle 120, it is possible to switch between the first state as a charging state in which the needle tip 121 of the inner needle 120 is located on more distal end side than the needle tip 111 of the outer needle 110 and a second state in which the needle tip 111 of the outer needle 110 is located on more distal end side than the needle tip 121 of the inner needle 120 as a result of sliding of the inner needle 120 and the outer needle 110 in the distal end direction. This configuration makes it possible to reliably collect the body tissue 2b into the notch portion 123 and the groove 124. Moreover, as described above, the biopsy needle 100 has the protruding length of the inner needle 120 at the time of biopsy formed to be shorter than the length in the conventional case. Accordingly, penetration of the capsule 2a of the prostate 2 from the inside to the outside with the needle tip 121 of the inner needle 120 is significantly less likely to occur.

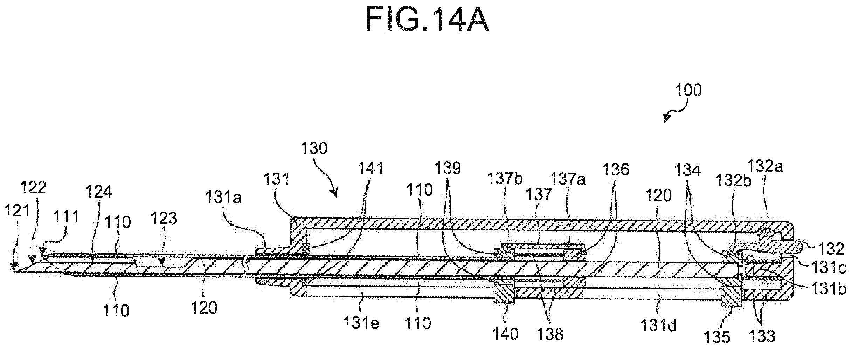

The biopsy operation of the biopsy needle 100 will be described in more detail. FIGS. 14A to 14F are diagrams for illustrating the biopsy operation of the biopsy needle 100 illustrated in FIG. 11, being a cross sectional view of the biopsy needle 100 taken along a plane passing through the center axis of the biopsy needle 100 in the longitudinal direction and passing through the center of the needle tip of the inner needle 120.

FIG. 14A is a diagram illustrating the first state of the biopsy needle 100 described above. As illustrated in FIG. 14A, in the first state, the inner needle fixing hook 132b is caught in the recess of the inner needle slider 134, and the outer needle fixing hook 137b is caught in the recess of the outer needle slider 139, and the inner needle charging coil spring 133 and the outer needle charging coil spring 138 are compressed and charging energy for extension.

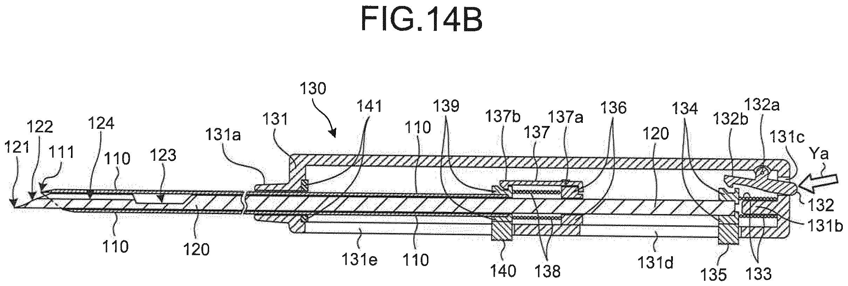

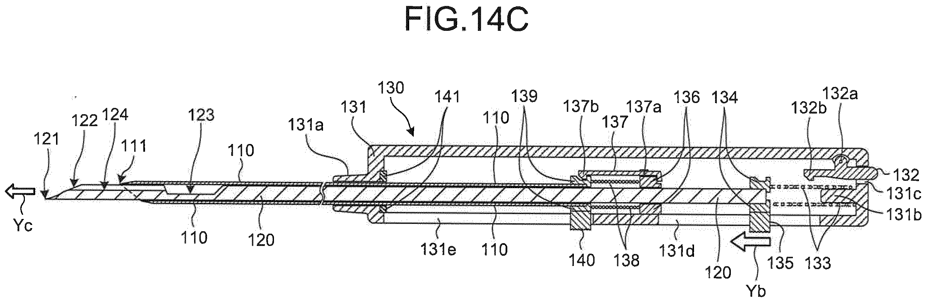

As illustrated by arrow Ya in FIG. 14B, when the trigger button 132 is pressed, the inner needle fixing hook 132b at the distal end portion is raised about the fulcrum 132a and disengaged from the recess of the inner needle slider 134. As illustrated in FIG. 14C, the compressed inner needle charging coil spring 133 extends in the distal end direction, and the inner needle slider 134 is biased by the extension of the inner needle charging coil spring 133 and slides in the distal end direction as indicated by arrow Yb. Together with this, the inner needle 120 also slides in the distal end direction as indicated by arrow Yc.

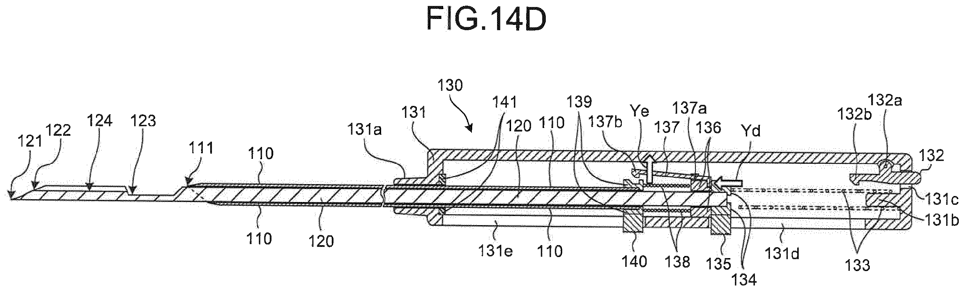

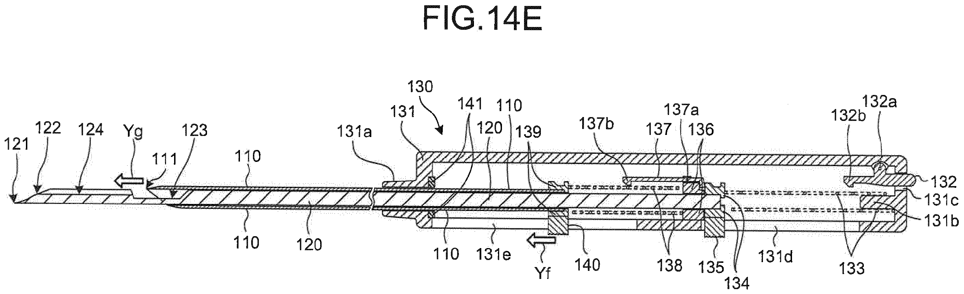

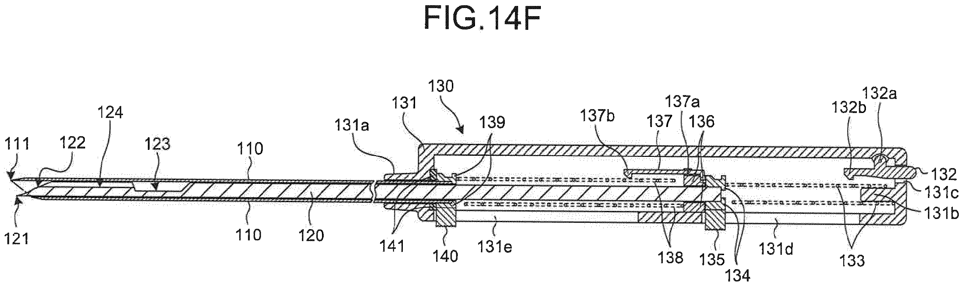

Then, as illustrated in FIG. 14D, the inner needle slider 134 and the inner needle knob 135 slide in the distal end direction by the biasing of the inner needle charging coil spring 133 until the inner needle slider 134 and the inner needle knob 135 abut the inner needle stopper 136. With this operation, the inner needle 120 protrudes in the x-axis direction by the stroke L₂. Furthermore, when the inner needle slider 134 presses the outer needle fixing hook release lever 137 from the proximal end as indicated by arrow Yd by the biasing of the inner needle charging coil spring 133, the outer needle fixing hook 137b at the distal end portion is raised about the fulcrum 137a as indicated by arrow Ye and disengaged from the recess of the outer needle slider 139. With this operation, as illustrated in FIG. 14E, the compressed outer needle charging coil spring 138 extends in the distal end direction. Biased by the extension of the outer needle charging coil spring 138, the outer needle slider 139 and the outer needle knob 140 move in the distal end direction until they abut the outer needle stopper 141 as illustrated by arrow Yf. With this configuration, as illustrated by arrow Yg, the outer needle 110 protrudes in the x-axis direction by the stroke L₁ while cutting the tissue that has entered the notch portion 123 by the needle tip 111, and then, the biopsy needle 100 is switched to the second state illustrated in FIG. 14F.

The operator of the biopsy needle 100 extracts the biopsy needle 100 from the biopsy site in this state. Subsequently, by moving the outer needle knob 140 to the proximal end of the outer needle knob groove 131e, the outer needle 110 is retreated (FIG. 14D), the body tissue inside the notch portion 123 is collected after the notch portion 123 is exposed. Thereafter, the inner needle knob 135 is moved to the proximal end of the inner needle knob groove 131d (FIG. 14A), thereby switching the biopsy needle 100 to the first state.

As described above, the biopsy needle 100 includes the moving mechanism in which the longitudinal stroke L₁ of the outer needle 110 is set to be longer than the longitudinal stroke L₂ of the inner needle 120 and configured to slidably move the outer needle 110 and the inner needle 120 independently in the longitudinal direction. With this configuration, it is possible to switch between the first state and the second state.

### (First Modification of First Embodiment)

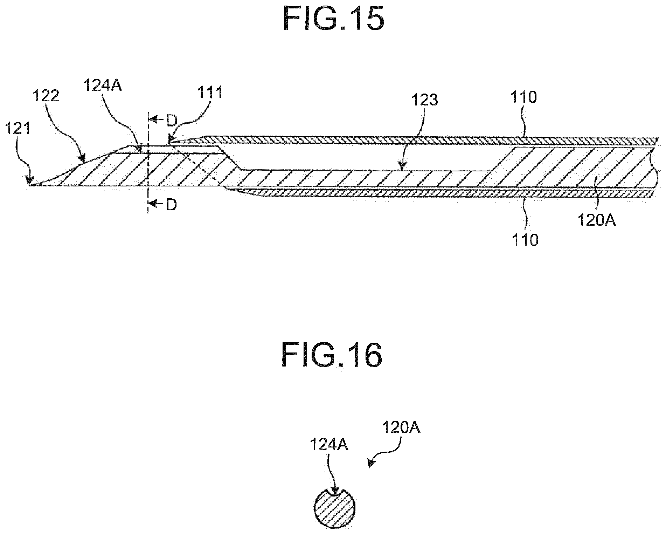

FIG. 15 is a cross-sectional view of a distal end section of a biopsy needle according to a first modification of the first embodiment, taken along a plane passing through the center axis of the biopsy needle in the longitudinal direction. FIG. 16 is a cross-sectional view taken along line D-D in FIG. 15. As illustrated by an inner needle 120A in FIGS. 15 and 16, a groove 124A that allows communication between the distal end portion and the notch portion 123 may have a semicircular cutout shape.

### (Second Modification of First Embodiment)

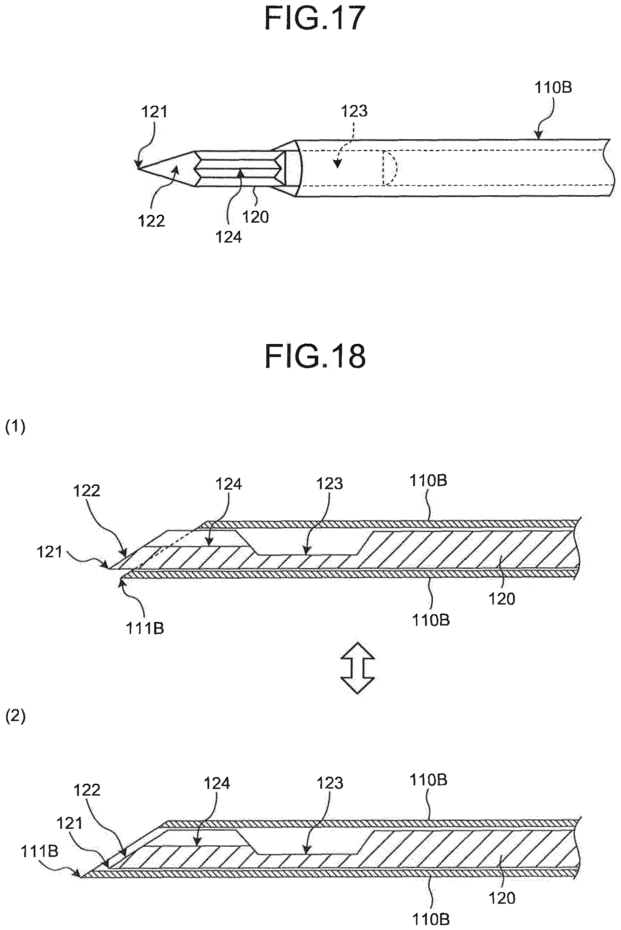

FIG. 17 is a plan view of the distal end section of the biopsy needle according to a second modification of the first embodiment. FIG. 18 is a cross-sectional view of the distal end section of the biopsy needle according to the second modification of the first embodiment, taken along a plane passing through the center axis of the biopsy needle in the longitudinal direction. (1) of FIG. 18 is a diagram illustrating the first state of the biopsy needle according to the second modification of the first embodiment, and (2) of FIG. 18 illustrates the second state of the biopsy needle according to the second modification of the first embodiment.

As illustrated in FIG. 17, an outer needle 110B may be turned upside down in the radial direction with respect to the inner needle 120. That is, the cutout at the distal end of the outer needle 110B and the inclined surface 123 may be parallel to each other. In this case, as illustrated in (1) of FIG. 18, the outer needle 110B does not need to cover the entire groove 124 in the first state as long as a needle tip 111P of the outer needle 110B reaches part of the inclined surface 122. That is, there is no problem with puncture of the biopsy needle into the body tissues as long as a region obtained by projecting the needle tip 111P of the outer needle 110B onto the transverse direction of the outer needle 110B and the inner needle 120 overlaps with the inclined surface 122 of the inner needle 120. Also in the second modification of the first embodiment, in the second state as illustrated in (2) of FIG. 18, there is a need to locate the needle tip 111P of the outer needle 110B on more distal end side than the needle tip 121 of the inner needle 120 to cover, with the outer needle 110B, the distal end of the groove 124 of the inner needle 120 and to reliably catch the body tissues into the notch portion 123 and the groove 124.

### (Second Embodiment)

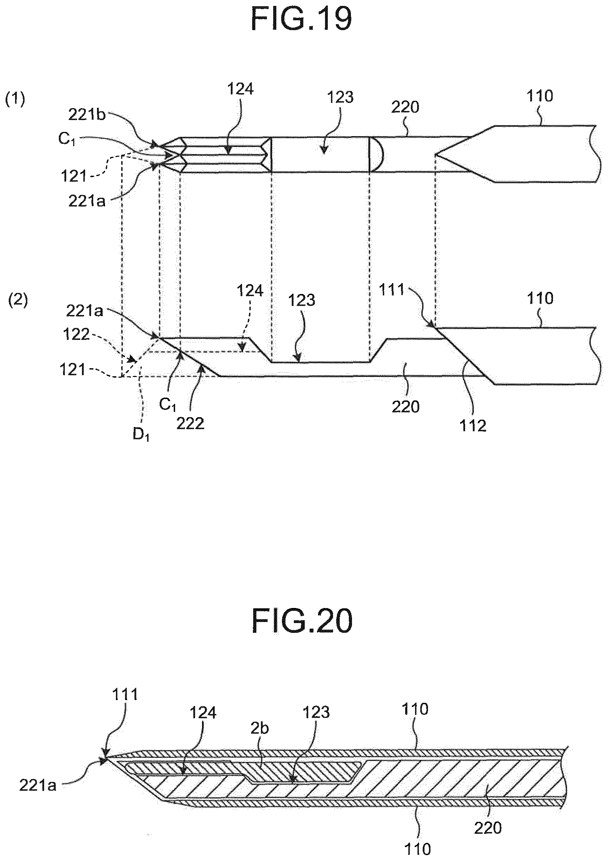

Next, a second embodiment will be described. FIG. 19 is a diagram for illustrating a distal end section of a biopsy needle according to the second embodiment. (1) of FIG. 19 is a plan view of the distal end section of the biopsy needle according to the second embodiment, and (2) of FIG. 19 is a side view of the distal end section of the biopsy needle according to the second embodiment. FIG. 20 is a cross-sectional view of the distal end section of biopsy needle according to the second embodiment after the body tissue collection, taken along a plane passing through the center axis of the biopsy needle in the longitudinal direction.

As illustrated in (1) of FIG. 19 and (2) of FIG. 19, the biopsy needle according to the second embodiment includes an inner needle 220 from which a distal end section D₁ of the inner needle 120A illustrated in FIG. 15 has been cut out. The inner needle 220 has a shape in which the distal end section D₁ of the inner needle 120A is cut out so that an inclined surface 222 is formed on a side opposite to the inclined surface 122 of the inner needle 120A. The inclined surface 222 is set such that the inclined surface 222 of the inner needle 220 and an inclined surface 112 of the outer needle 110 are substantially parallel to each other. Therefore, the needle tip of the inner needle 220 is divided into two needle tips 221a and 221b from a position C₁.

Since the inclined surface 222 of the inner needle 220 and the inclined surface 112 of the outer needle 110 are substantially parallel to each other, as illustrated in FIG. 20, it is possible to store the needle tips 221a and 221b of the inner needle 220 in the outer needle 110 merely by causing the needle tip 111 of the outer needle 110 to slightly protrude to the distal end side of the needle tip 221a of the inner needle 220. Moreover, the groove 124 is formed in the inner needle 220 so as to communicate with the notch portion 123 from the position C₁ between the needle tips 221a and 221b. Therefore, the body tissue 2b can be collected by substantially the distal end of the inner needle 220, and it is also possible to collect the body tissue in a state where the needle tips 221a and 221b of the inner needle 220 do not protrude from the body tissue collection target region. Since the needle tips 221a and 221b of the inner needle 220 can also be stored inside the outer needle 110 merely by causing the needle tip 111 to slightly protrude to the distal end side of the needle tip 221a of the inner needle 220. Accordingly, the needle tip 111 of the outer needle 110 has substantially no protrusion from the body tissue collection target region, and thus, there is no possibility of damaging a section outside the collection target region.

In the second embodiment, the groove formed in the inner needle is not limited to the V-shaped groove 124, but may be a groove 124A (refer to FIG. 16) in a semicircular cutout shape.

### (First Modification of Second Embodiment)

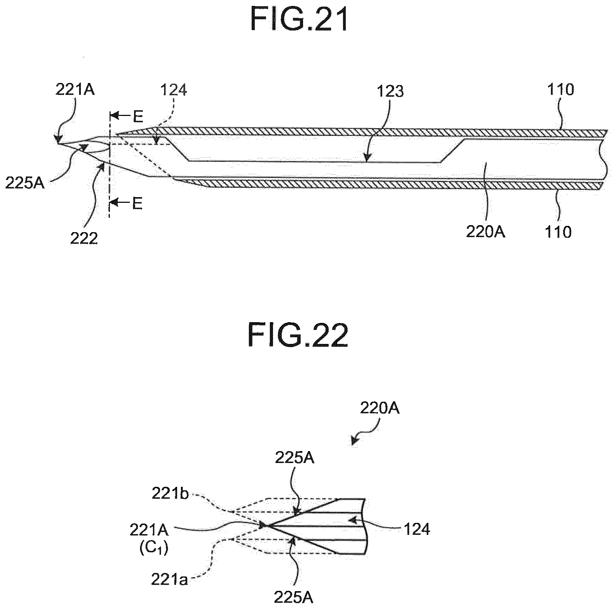

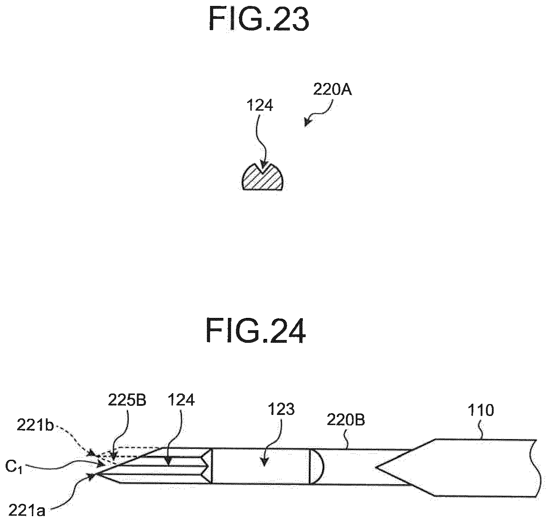

FIG. 21 is a diagram for illustrating the distal end section of the biopsy needle according to a first modification of the second embodiment. FIG. 21 illustrates, for the purpose of explanation, the outer needle in a cross-section taken along a plane passing through the center axis of the outer needle in the longitudinal direction. FIG. 22 is a perspective view of the distal end portion of the inner needle illustrated in FIG. 21. FIG. 23 is a cross-sectional view taken along line E-E in FIG. 21.

As illustrated by an inner needle 220A in FIGS. 21 to 23, D cutting is performed on two surfaces 225A such that a needle tip 221A is formed at the position C₁, and then, a section including the needle tips 221a and 221b of the inner needle 220 illustrated in FIG. 19 are cut off. With this shape, the groove 124 directly leads to the notch portion 123 from the needle tip 221A (position C₁), and thus, the body tissue 2b can be collected by the most distal end of the inner needle 220A. Accordingly, it would be sufficient with substantially no protrusion of the inner needle 220A and the outer needle 110 from the body tissue collection target region. Moreover, the configuration of the inner needle 220A with the needle tip that is not divided into two reduces the resistance at the time of puncture, and achieves easy transmission of the force of puncture to the needle tip 221A, making it possible to perform puncture with less force.

FIG. 24 is a plan view of the distal end section of another biopsy needle according to the first modification of the second embodiment. As an inner needle 220B illustrated in FIG. 24, it is also possible to adopt a shape obtained by cutting off a needle tip 221b section of the inner needle 220 illustrated in FIG. 19 by D cutting performed on a surface 225B Since this shape of the inner needle 220B with the needle tip is not divided into two, the resistance at the time of puncture is reduced, and easy transmission of the force of puncture to the needle tip 221a is achieved, making it possible to perform puncture with less force.

### (Second Modification of Second Embodiment)

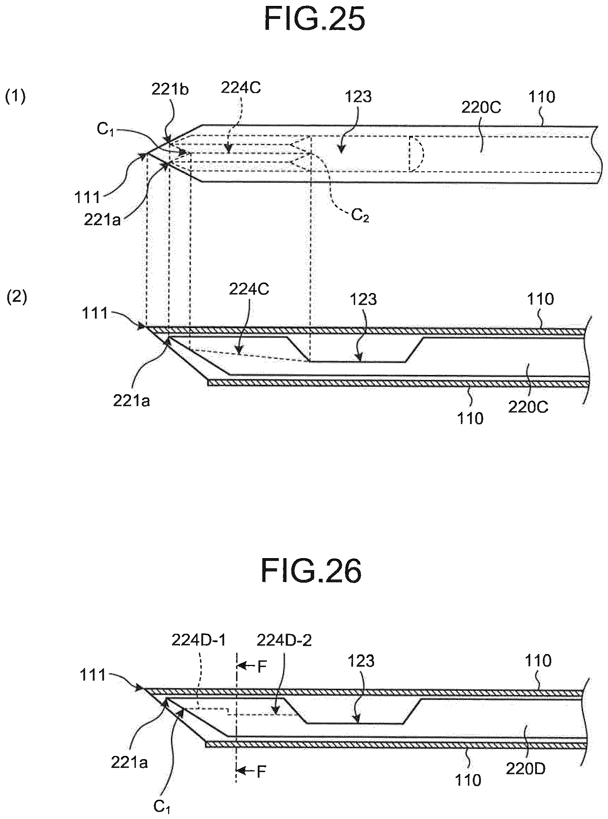

Next, second modification the second embodiment will be described. FIG. 25 is a diagram for illustrating a distal end section of a biopsy needle according to a second modification of the second embodiment. (1) of FIG. 25 is a plan view of the distal end section of the biopsy needle according to the second modification of the second embodiment, and (2) of FIG. 25 is a side view of the distal end section of the biopsy needle according to the second modification of the second embodiment. For the purpose of explanation, the outer needle is illustrated in a cross-sectional state taken along a plane passing through the center axis of the outer needle in the longitudinal direction. A groove 224C of an inner needle 220C in FIG. 25 is formed such that the bottom of the groove 224C is inclined from the position C₁ of the distal end portion toward a center C₂ of a connecting portion with the notch portion 123, as compared with the groove 124 of the inner needle 220 illustrated in FIG. 19. In other words, the groove 224C is formed so that its depth becomes shallower toward the distal end portion. In this case, the protrusion of the inner needle 220C allows the body tissue to enter the groove 224C in a state where the needle tip side is thinner than the notch portion 123 side. This configuration makes it is easier to perform cutting by the distal end of the outer needle 110 and makes it possible to reliably gather the body tissue into the grooves 224C and the notch portion 123. Of course, similarly to the second embodiment, the body tissue can be collected by the substantially distal end of the inner needle 220C.

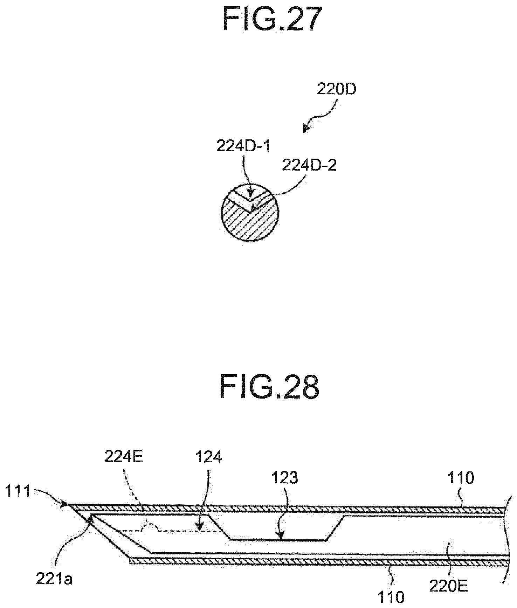

FIG. 26 is a diagram illustrating another example of the distal end section of the biopsy needle according to the second modification of the second embodiment. FIG. 26 illustrates, for the purpose of explanation, the outer needle in a cross-section taken along a plane passing through the center axis of the outer needle in the longitudinal direction. FIG. 27 is a sectional view taken along line F-F in FIG. 26.

As illustrated by an inner needle 220D in FIGS. 26 and 27, it is allowable to provide a step on the bottom of the groove such that a groove 224D-1 on the distal end portion side is shallower than a groove 224D-2 on the connecting portion side with the notch portion 123. Also in this case, the body tissue enters the grooves 224D-1, and 224D-2 in a state where the needle tip side is thinner than the notch portion 123 side, making it easier to perform cutting by the distal end of the outer needle 110.

FIG. 28 is a diagram illustrating another example of the distal end section of the biopsy needle according to the second modification of the second embodiment. Similarly, FIG. 28 illustrates, for the purpose of explanation, the outer needle in a cross-section taken along a plane passing through the center axis of the outer needle in the longitudinal direction. As in the inner needle 220E illustrated in FIG. 28, a protrusion 224E may be provided in the middle of the groove 124. With this protrusion 224E, the body tissue that has entered the groove 124 becomes thinner in the middle, achieving smooth cutting by the distal end of the outer needle 110. Since the body tissue is likely to be cut at the position of the protrusion 224E, it is preferable to arrange the protrusion 224E on more distal end side rather than the proximal end side of the groove 124 in order to increase the amount of body tissue to be collected in the groove 124.

### (Third Embodiment)

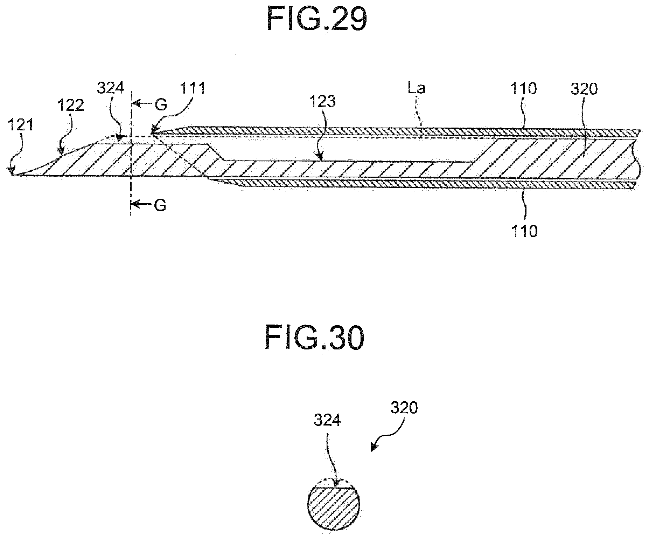

Next, a third embodiment will be described. FIG. 29 is a cross-sectional view of a distal end section of the biopsy needle according to the third embodiment, taken along a plane passing through the center axis of the biopsy needle in the longitudinal direction. In FIG. 29, the distal end shape of the inner needle before formation of the cutout portion is indicated by broken line La. FIG. 30 is a cross sectional view taken along line G-G in FIG. 29.

As illustrated in FIGS. 29 and 30, in the third embodiment, the inner needle 320 includes a cutout portion 324 (guide portion) formed instead of the groove 124 illustrated in FIG. 1. The cutout portion 324 is formed by cutting out a section from the proximal end of the inclined surface 122 of the distal end portion to the notch portion 123 in the longitudinal direction. Even in a case where the cutout portion 324 is formed, body tissue naturally enters the cutout portion 324 by energy at the time of ejection of the inner needle 320 when the body tissue is punctured. Subsequently, by allowing the inner needle 320 to further eject, the body tissue is introduced to the notch portion 123 via the cutout portion 324. Therefore, even in the case where the cutout portion 324 is formed, as long as cutout portion 324 reaches the body tissue collection target region, the cutout portion 324 guides entry of body tissue into the notch portion 123, making it possible to accommodate the collection target body tissue in the notch portion 123.

### (First Modification of Third Embodiment)

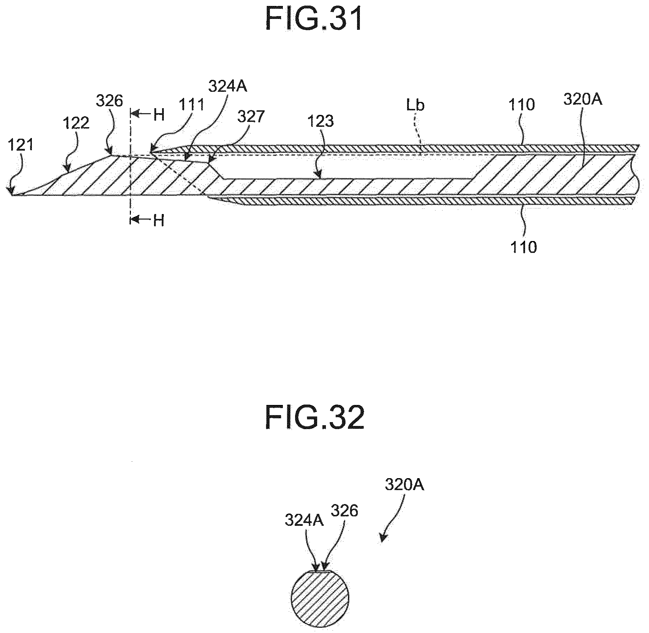

Next, a first modification the third embodiment will be described. FIG. 31 is a cross-sectional view of the distal end section of the biopsy needle according to the first modification of the third embodiment, taken along a plane passing through the center axis of the biopsy needle in the longitudinal direction. In FIG. 31, the distal end shape of the inner needle before forming the cutout portion is indicated by broken line Lb. FIG. 32 is a cross-sectional view taken along line H-H in FIG. 31. As illustrated in an inner needle 320A in FIGS. 31 and 32, a cutout surface of a cutout portion 324A may be inclined from a proximal end 326 of the inclined surface 122 as the distal end portion toward a connecting portion 327 with the notch portion 123. Even in this case, similarly to the groove 224C of the inner needle 220C illustrated in FIG. 25, a gap between the inner needle 320A and the outer needle 110 becomes narrower toward the distal end portion. This makes it easier to perform cutting by the distal end of the outer needle 110, making it possible to reliably gather the body tissue into the cutout portion 324A and the notch portion 123.

### (Second Modification of Third Embodiment)

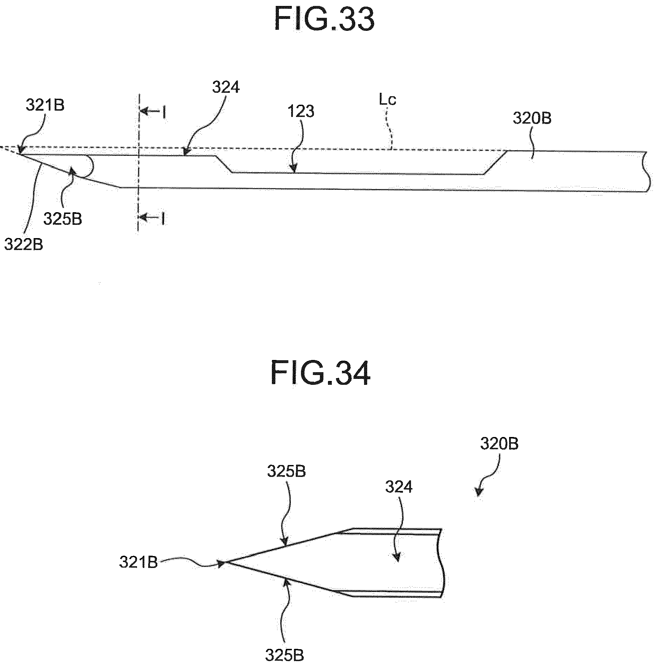

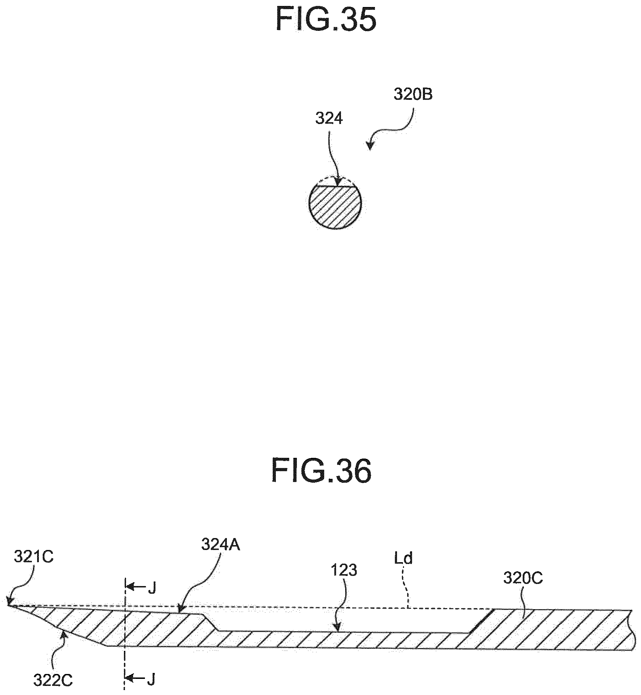

Next, a second modification of the third embodiment will be described. FIG. 33 is a side view of the distal end section of the inner needle according to the second modification of the third embodiment. In FIG. 33, the distal end shape of the inner needle before forming the cutout portion is indicated by broken line Lc. FIG. 34 is a perspective view of the distal end section of the inner needle illustrated in FIG. 33. FIG. 35 is a cross-sectional view taken along line I-I in FIG. 33.

While the inner needle 320 illustrated in FIG. 29 has a configuration in which the notch portion 123 and the cutout portion 324 are located on the side different from the needle tip 121 at the distal end of the inclined surface 122 in the transverse direction, an inner needle 320B according to the second modification of the third embodiment illustrated in FIGS. 33 to 35 is configured to arrange the notch portion 123 and the cutout portion 324 on the same side as a needle tip 321B at the distal end of an inclined surface 322B in the transverse direction. With this shape, the cutout portion 324 starts directly from the needle tip 321B, and thus, the body tissue can be collected by the distal end of the inner needle 320B. Accordingly, it would be sufficient with substantially no protrusion of the inner needle 320B and the outer needle 110 from the body tissue collection target region. Furthermore, the inner needle 320B is formed such that the side of the needle tip 321B undergoes D cutting from both sides on a surface 325B to taper the needle tip 321B, achieving easy transmission of the force of puncture to the needle tip 321B.

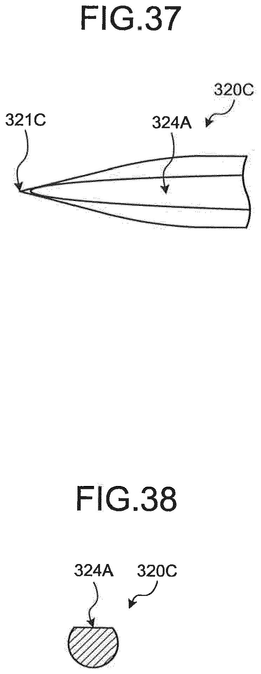

FIG. 36 is a cross-sectional view of a distal end section of another inner needle according to the second modification of the third embodiment taken along a plane passing through the center axis of the inner needle in the longitudinal direction. FIG. 37 is a perspective view of the distal end section of the inner needle illustrated in FIG. 36. FIG. 38 is a sectional view taken along line J-J in FIG. 36. While the inner needle 320A illustrated in FIG. 31 has a configuration in which the notch portion 123 and the cutout portion 324A are positioned on the side different from the needle tip 121 formation portion (top portion) at the distal end of the inclined surface 122 in the transverse direction, an inner needle 320C illustrated in FIGS. 36 to 38 is configured to arrange the notch portion 123 and the cutout portion 324A on the same side as a formation portion of a needle tip 321C at the distal end of an inclined surface 322C in the transverse direction. Also in this case, the cutout portion 324A leads directly from the needle tip 321C to the notch portion 123, and thus, the body tissue can be collected by the distal end of the inner needle 320C. Accordingly, it would be sufficient with substantially no protrusion of the inner needle 320C and the outer needle 110 from the body tissue collection target region. In the inner needle 320C, since the cutout surface of the cutout portion 324A has a shape that is inclined from the needle tip 321C toward the proximal end, the distal end of the inner needle 320C is not cut out. Accordingly, the needle tip 321C can maintain the pointed shape and there is no need to perform D cutting on the side surface of the distal end portion.

### (Fourth Embodiment)

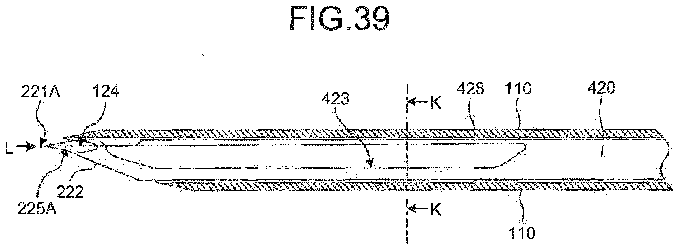

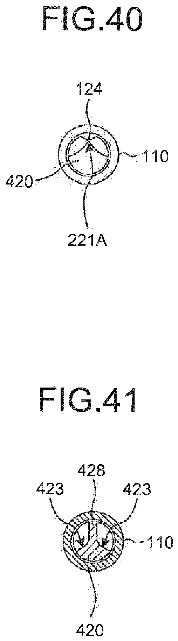

Next, a fourth embodiment will be described. FIG. 39 is a diagram for illustrating a distal end section of a biopsy needle according to the fourth embodiment. FIG. 39 illustrates, for the purpose of explanation, the outer needle in a cross-section taken along a plane passing through the center axis of the outer needle in the longitudinal direction. FIG. 40 is a schematic view in the direction of arrow L in FIG. 39. FIG. 41 is a cross-sectional view taken along line K-K in FIG. 39.

In comparison with the inner needle 220A in FIGS. 21 to 23, an inner needle 420 of the fourth embodiment illustrated in FIGS. 39 to 41 is configured to include a rib 428 provided in a notch portion 423 to reinforce the inner needle strength. With this configuration, the inner needle 420 achieves easier transmission of the puncture force for the needle tip 221A, making it possible to puncture harder tissue. In addition, it is also possible to extend the notch portion 423 further to the distal end, compared with the inner needle 220A in FIGS. 21 to 23.

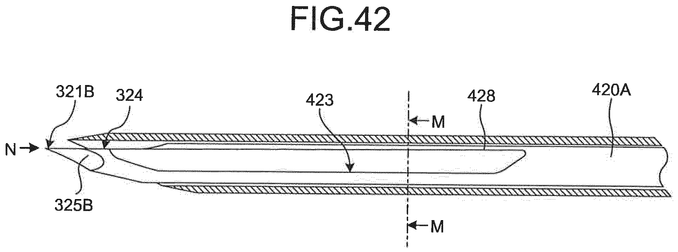

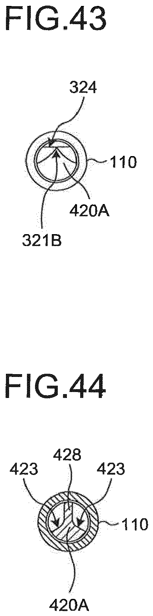

FIG. 42 is a diagram for illustrating a distal end section of another biopsy needle according to the fourth embodiment. FIG. 42 illustrates, for the purpose of explanation, the outer needle in a cross-section taken along a plane passing through the center axis of the outer needle in the longitudinal direction. FIG. 43 is a schematic view in the direction of arrow N in FIG. 42. FIG. 44 is a cross-sectional view taken along line M-M in FIG. 42. For the inner needle 320B illustrated in FIGS. 33 to 35, as the case of an inner needle 420A illustrated in FIGS. 42 to 44, it is also allowable to provide the rib 428 in the notch portion 423 to reinforce the inner needle strength, thereby further extending the notch portion 423 to the distal end side.

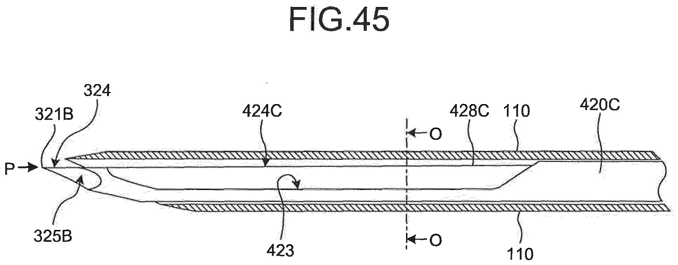

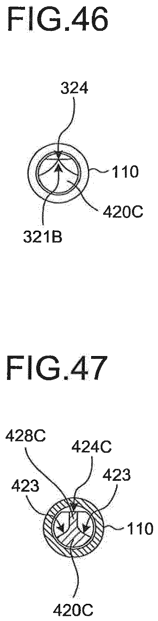

FIG. 45 is a diagram for illustrating a distal end section of another biopsy needle according to the fourth embodiment. FIG. 45 illustrates, for the purpose of explanation, the outer needle in a cross-section taken along a plane passing through the center axis of the outer needle in the longitudinal direction. FIG. 46 is a schematic view in the direction of arrow P in FIG. 45. FIG. 47 is a sectional view taken along line O-O in FIG. 45. As illustrated in an inner needle 420C in FIGS. 45 to 47, it is allowable to extend the cutout portion 324 at the distal end of the inner needle 420A illustrated in FIGS. 42 to 44 and to form a cutout portion 424C obtained by cutting out an upper portion of a rib 428C from distal end to the proximal end of the rib 428C. According to the inner needle 420C with this configuration, it is possible to increase the amount of body tissue to be collected by the amount of formation of the cutout portion 424C, enabling communication between the notch portions 423 partitioned by the rib 428C with the cutout portion 424C. Accordingly, it is possible to take out almost all of the collected body tissue from one of the notch portions 423, leading to achieving an effect of reducing the number of times of operation of taking out the body tissue from the biopsy needle to once.

Although the tissue of the prostate 2 is collected using the biopsy needle 100 in the embodiments, the biopsy needle 100 may also be used to collect tissues of any other site. Moreover, although the distal end of the biopsy needle 100 is allowed to reach the biopsy site via the treatment tool channel of the insertion unit 10 of the ultrasound endoscope in the embodiments, it is possible to perform puncture by the biopsy needle 100 from outside of the body without going through the treatment tool channel of the ultrasonic endoscope, depending on the biopsy site.

### Industrial Applicability

As described above, a biopsy needle according to the present invention is useful for reliably collecting tissues and reducing a length of the biopsy needle protruding from a tissue collection target site at the time of collecting the tissues.

### Reference Signs List

2 PROSTATE
2a CAPSULE
3 URETHRA
4 BLADDER
10 INSERTION UNIT
11 OPENING
100, 100P, 100P' BIOPSY NEEDLE
110, 110B, 110P OUTER NEEDLE
111, 111P, 121, 121P, 221a, 221b, 221A, 321B, 321C NEEDLE TIP
112, 122, 122P, 122P', 222, 322B, 322C INCLINED SURFACE
120, 120A, 120P, 120P', 220, 220A, 220B, 220C, 220D, 220E, 320, 320A, 320B, 320C, 420, 420A, 420C INNER NEEDLE
123, 123P, 123P', 423 NOTCH PORTION
124, 124A, 224C, 224D-1, 224D-2 GROOVE
130 OPERATING UNIT
131 OPERATING UNIT MAIN UNIT
131a LUER FITTING PORTION
131b SPRING ASSEMBLY PROTRUSION
131c TRIGGER BUTTON HOLE
131d INNER NEEDLE KNOB GROOVE
131e OUTER NEEDLE KNOB GROOVE
132 TRIGGER BUTTON
132a, 137a FULCRUM
132b INNER NEEDLE FIXING HOOK
133 INNER NEEDLE CHARGING COIL SPRING
134 INNER NEEDLE SLIDER
135 INNER NEEDLE KNOB
136 INNER NEEDLE STOPPER
137 OUTER NEEDLE FIXING HOOK RELEASE LEVER
137b OUTER NEEDLE FIXING HOOK
138 OUTER NEEDLE CHARGING COIL SPRING
139 OUTER NEEDLE SLIDER
140 OUTER NEEDLE KNOB
141 OUTER NEEDLE STOPPER
224 PROJECTION E
324, 324A, 424C CUTOUT PORTION
428, 428C RIB

## Claims

1. A biopsy needle comprising:
an outer needle being tubular and having a first needle tip at one end of the outer needle in a longitudinal direction; and
an inner needle having a pillar shape and configured to be inserted into and movable in the outer needle in the longitudinal direction of the outer needle, the inner needle comprising:
a distal end portion having a second needle tip at a distal end of the inner needle and having an inclined surface inclined toward the second needle tip at the distal end;
a notch portion having a notch on a side surface closer to a proximal end side of the inner needle than the inclined surface to collect body tissues; and
a guide portion leading from the distal end portion to the notch portion.

2. The biopsy needle according to claim 1, wherein
the guide portion is a groove leading from the distal end portion to the notch portion.

3. The biopsy needle according to claim 2, wherein
a depth of the groove is shallower at the distal end portion than at a connection between the guide portion and the notch portion.

4. The biopsy needle according to claim 3, wherein
a bottom of the groove is inclined from the distal end portion toward the connection.

5. The biopsy needle according to claim 1, wherein
the guide portion is a cutout portion extending from the distal end portion to the notch portion in a longitudinal direction of the inner needle.

6. The biopsy needle according to claim 5, wherein
a cutout surface of the cutout portion is inclined from the distal end portion toward the connection.

7. The biopsy needle according to claim 1, wherein
the notch portion has a rib.

8. The biopsy needle according to claim 7, wherein
an upper portion of the rib is cut out in a longitudinal direction of the inner needle.

9. The biopsy needle according to claim 1, wherein
the notch portion and the guide portion are located on a same side as the second needle tip in a transverse direction of the inner needle.

10. The biopsy needle according to claim 1, wherein
the notch portion and the guide portion are located on an opposite side to the second needle tip in a transverse direction of the inner needle.

11. The biopsy needle according to claim 1, wherein
a distal end of the outer needle is obliquely cut out to form the first needle tip, and
the inclined surface of the inner needle and a cutout at the distal end of the outer needle are substantially parallel to each other.

12. The biopsy needle according to claim 1, further comprising a moving mechanism configured to slide the outer needle and the inner needle in the longitudinal direction independently of each other such that a maximum slidable distance of the outer needle in a distal end direction is larger than a maximum slidable distance of the inner needle in a distal end direction,
wherein the moving mechanism is configured to move the outer needle and the inner needle so as to switch between a first state in which the second needle tip is located on more distal end side than the first needle tip and a second state in which the first needle tip is located on more distal end side than the second needle tip.

13. The biopsy needle according to claim 1, wherein
the inner needle is columnar.
